# EUROPEAN PATENT APPLICATION

(11) **EP 1 917 922 A1**
(43) Date of publication of application: **07.05.2008**
(21) Application number: 07020299.9
(22) Date of filing: 17.10.2007
(51) Int. Cl.: A61B 17/22, A61F 2/84

(54) **Stent recovery apparatus**

(30) Priority: 02.11.2006 JP 2006298817
(71) Applicant: OLYMPUS MEDICAL SYSTEMS CORP., Shibuya-ku Tokyo 151-0072 (JP)
(72) Inventor: Goto, Hiroaki, Shibuya-ku Tokyo 151-0072 (JP)
(74) Representative: von Hellfeld, Axel

(57) **Abstract**

A stent recovery apparatus that recovers a stent which is left inside a body cavity and which comprises a hollow drum portion which is formed by a single wire or a stranded wire which has a free end, and which is disassembled by the wire or the stranded wire being unwound by being pulled, the stent recovery apparatus including: a narrow elongated traction tool that catches the free end of the wire or stranded wire of the stent; a through portion through which the traction tool and the wire or the stranded wire can be retrievably inserted, and whose distal end can be placed against the drum portion; and a retrieval tool that retrieves the wire or the stranded wire which has been inserted through the through portion together with the traction tool.

## Description

### BACKGROUND OF THE INVENTION

### Field of the Invention

The present invention relates to a stent recovery apparatus for recovering stent which has been retained in a body cavity.

Priority is claimed on Japanese Patent Application No. 2006-298817, filed November 2, 2006, the contents of which are incorporated herein by reference.

### Description of the Related Art

When a hollow organ is constricted, in order to protect the lumen of the hollow organ, a stent is retained in the constricted portion. Such stents include those that are left for an extended time period in the hollow organ, and those that are only left for a predetermined period and are recovered and removed once the viability of the lumen has been secured. Various recovery apparatuses have been proposed in order to recover the stent after a fixed period (see, for example, Japanese Unexamined Patent Application, First Publication (JP-A) Nos. 2006-55330 and 2001-204826).

However, when recovering a stent that has only been used for a predetermined period from a hollow organ such as an esophagus using the aforementioned conventional stent recovery apparatus, the stent is pulled through a sheath and is recovered while being deformed. Accordingly, it is difficult to extract the stent easily.

### SUMMARY OF THE INVENTION

The present invention was conceived in view of the above described circumstances, and it is an object thereof to provide a stent recovery apparatus that makes it possible to efficiently and quickly recover a stent.

In order to solve the above described problems, the present invention employs the following means.

The stent recovery apparatus of the present invention recovers a stent which is left inside a body cavity and which comprises a hollow drum portion which is formed by a single wire or a stranded wire which has a free end, and which is disassembled by the wire or the stranded wire being unwound by being pulled, the stent recovery apparatus including: a narrow elongated traction tool that catches the free end of the wire or stranded wire of the stent; a through portion through which the traction tool and the wire or the stranded wire can be retrievably inserted, and whose distal end can be placed against the drum portion; and a retrieval tool that retrieves the wire or the stranded wire.

This invention makes it possible to unwind the drum portion of a stent and restore the stent to the form of a single wire or stranded wire by pulling the free end of the wire or stranded wire of the drum portion using a traction tool. In addition, by gripping the wire or stranded wire using a traction tool, and retrieving the wire or stranded wire using a retrieval tool while the insertion portion is placed against the drum portion, the stent can be recovered to the outside of the body while the drum portion of the stent is being unwound.

In the stent recovery apparatus according to the present invention, the through portion may be formed in a tube shape, and a base end of the through portion may be connected to the retrieval tool thereby connecting together the through portion and the retrieval tool.

This invention makes it possible when a stent is being recovered for direct handling of the moving wire or stranded wire by an operator to be restricted to a favorable minimum because the wire or stranded wire moves through the through portion.

In the stent recovery apparatus according to the present invention, the retrieval tool may include: a shaft portion around which the wire or stranded wire is wound; and a cover portion that covers at least one end side of the shaft portion.

This invention makes it possible when a stent is being recovered for direct handling of the moving wire or stranded wire by an operator to be restricted to a favorable minimum because the shaft portion around which the wire or stranded wire is wound is housed inside a cover portion.

In the stent recovery apparatus according to the present invention, the stent recovery apparatus may further include an engaging portion that engages the retrieval tool with a base end of the traction tool.

This invention makes it possible when the wire or stranded wire is being retrieved using a retrieval tool for the direction in which the traction tool is retrieved to be fixed relative to the retrieval tool, so that the traction tool can be retrieved smoothly.

In the stent recovery apparatus according to the present invention, the stent recovery apparatus may further include an endoscope that has an insertion portion in which a treatment tool through channel is provided, and the through portion may be formed by the treatment tool through channel.

This invention makes it possible for a stent to be recovered by inserting a wire or stranded wire directly through a treatment tool through channel of an endoscope.

According to the present invention, it is possible to efficiently and quickly recover a stent.

### BRIEF DESCRIPTION OF THE DRAWINGS

FIG 1 is a schematic view showing a stent recovery apparatus according to a first embodiment of the present invention.
FIG 2 is a perspective view showing a stent recovered using the stent recovery apparatus according to the first embodiment of the present invention.
FIG. 3 is a perspective view showing a variant example of the stent recovered using the stent recovery apparatus according to the first embodiment of the present invention.
FIG 4 is a schematic view showing the stent recovery apparatus according to the first embodiment of the present invention which recovers the stent shown in FIG. 3.
FIG 5 is a plan view showing a retrieval tool of the stent recovery apparatus according to the first embodiment of the present invention.
FIG. 6 is a side view showing a retrieval tool of the stent recovery apparatus according to the first embodiment of the present invention.
FIG 7 is a plan view showing a side aperture in a cover portion of the retrieval tool of the stent recovery apparatus according to the first embodiment of the present invention.
FIG. 8 is a plan view showing a variant example of a side aperture in the cover portion of the retrieval tool of the stent recovery apparatus according to the first embodiment of the present invention.
FIG 9 is a view illustrating the action of the stent recovery apparatus according to the first embodiment of the present invention.
FIG 10 is a view illustrating the action of the stent recovery apparatus according to the first embodiment of the present invention.
FIG 11 is a view illustrating the action of the stent recovery apparatus according to the first embodiment of the present invention.
FIG 12 is a view illustrating the action of the stent recovery apparatus according to the first embodiment of the present invention.
FIG 13 is a view illustrating the action of the stent recovery apparatus according to the first embodiment of the present invention.
FIG 14 is a view illustrating the action of the stent recovery apparatus according to the first embodiment of the present invention.
FIG 15 is a view illustrating the action of the stent recovery apparatus according to the first embodiment of the present invention.
FIG 16 is a view illustrating the action of the stent recovery apparatus according to the first embodiment of the present invention.
FIG 17 is a view illustrating the action of the stent recovery apparatus according to the first embodiment of the present invention.
FIG 18 is a schematic view showing a stent recovery apparatus according to a second embodiment of the present invention.
FIG 19 is a side view showing a retrieval tool of the stent recovery apparatus according to the second embodiment of the present invention.
FIG 20 is a plan view showing the stent recovery apparatus according to the second embodiment of the present invention.
FIG. 21 is a view illustrating the action of the stent recovery apparatus according to the second embodiment of the present invention.
FIG 22 is a view illustrating the action of the stent recovery apparatus according to the second embodiment of the present invention.
FIG 23 is a schematic view showing a variant example of the stent recovery apparatus according to the first embodiment of the present invention.
FIG 24 is a schematic view showing a stent recovery apparatus according to another embodiment of the present invention.
FIG 25 is a view illustrating the action of the stent recovery apparatus shown in FIG 24.
FIG 26 is a schematic view showing a variant example of the stent recovery apparatus shown in FIG 24.

### DETAILED DESCRIPTION OF THE INVENTION

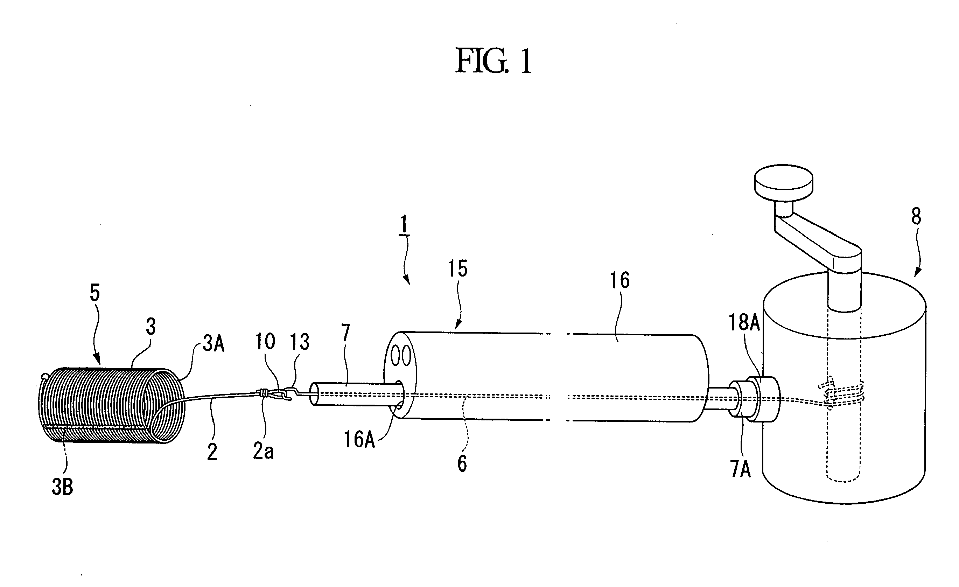

The first embodiment of the present invention will now be described with reference made to FIGS. 1 through 17.

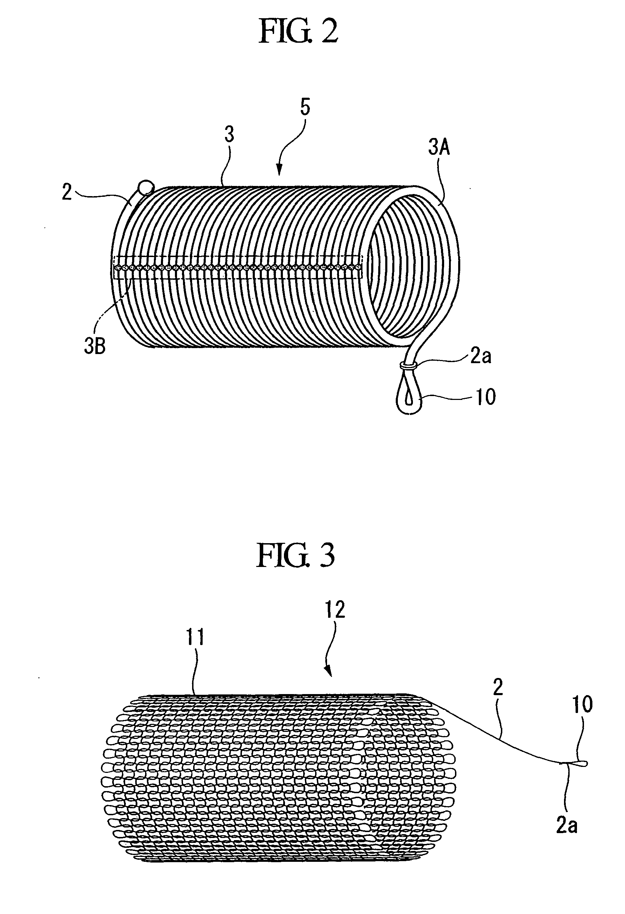

As is shown in FIGS. 1 and 2, the stent recovery apparatus 1 according to the present embodiment recovers a stent 5 that is left inside a body cavity, and comprises a hollow drum portion 3 in which a single wire 2 which has a free end 2a is wound around so as to form substantially circular loops 3A, and which is disassembled by the wire 2 being unwound. The stent recovery apparatus 1 comprises a narrow elongated traction wire (i.e., a traction tool) 6 that catches the free end 2a of the stent 5, a sheath (i.e., a through portion) 7 which is formed in a tube shape and which is placed against the drum portion 3 of the stent 5 and through which the wire 2 is inserted, and a retrieval tool 8 that, when a distal end of the sheath 7 is placed against the stent 5, retrieves the wire 2 which has been inserted through the sheath 7 together with the traction wire 6.

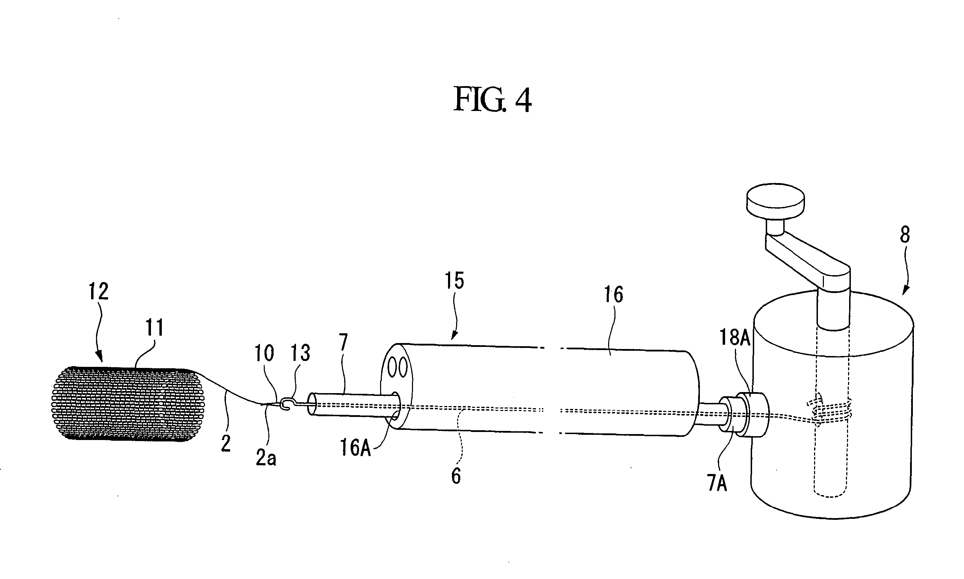

Each loop 3A of the drum portion 3 of the stent 5 is adhered or thermally fused together by a connecting portion 3B. A loop-shaped catching portion 10 with which a hook 13 (described below) which is formed in the traction wire 6 engages is formed in the free end 2a of the stent 5. Note that as is shown in FIG 3 and FIG. 4, as in the case of a stent 12 that has a drum portion 11 in which the wire 2 is flat-woven, the structure of the stent is not particularly restricted provided that the wire 2 can be unwound when the free end 2a is pulled. Moreover, the material of the stent 5 is not limited to the wire 2 and a single stranded wire may also be employed.

As is shown in FIG 1, a hook 13 that engages with the catching portion 10 of the stent 5 is provided at the distal end of the traction wire 6.

The sheath 7 comprises an inner diameter that allows the wire 2 to be inserted through it, and an outer diameter that allows it to be retractably inserted into a treatment tool through channel 16A which is provided in an insertion portion 16 of an endoscope 15. A sheath side aperture 7A that has a larger diameter than the sheath 7 and is connected to the retrieval tool 8, and that links together the sheath 7 and the retrieval tool 8 is provided in a base end of the sheath 7. The length of the sheath 7 is made shorter than the length of the traction wire 6 such that the hook 13 of the traction wire 6 is able to protrude from the distal end of the sheath 7.

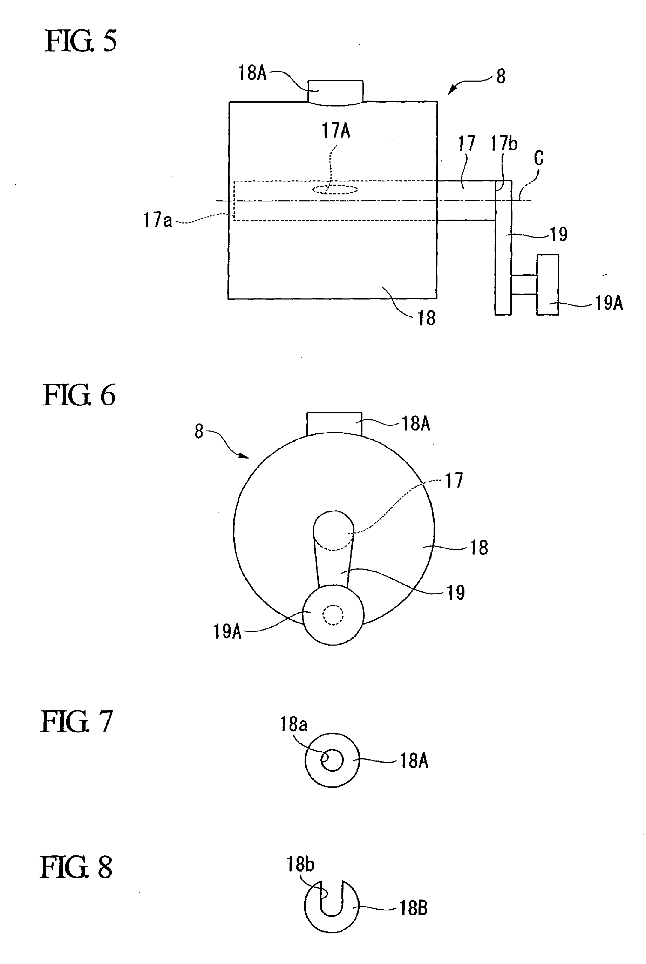

As is shown in FIG. 5 and FIG 6, the retrieval tool 8 comprises a shaft portion 17 around which the wire 2 is wound, a cover portion 18 which covers at least a one end 17A side of the shaft portion 17, and a rotation operating portion 19 which is connected to an other end 17b of the shaft portion 17 and which rotates the shaft portion 17 around a center axis C.

An engaging hole 17A through which the base end of the traction wire 6 is inserted and is then engaged is provided at a position on the same axis as a cover side aperture 18A of the cover portion 18 (described below) in the one end 17a side of the shaft portion 17 which is housed within the cover portion 18. Note that it is also possible for the entire shaft portion to be housed within the cover portion such that only the rotation operating portion 19 is exposed from the cover portion 18.

It is preferable for the cover portion 18 to be constructed from a transparent material so that the interior thereof is visible.

A cover side aperture 18A that is connected to the sheath side aperture 7A and that links together the sheath 7 and the cover portion 18 is provided in a side surface of the cover portion 18. As is shown in FIG 7, a through hole 18a which is large enough to allow the sheath 7 to pass therethrough is provided in the cover side aperture 18A.

Note that it is also possible to construct a cover portion in which the one end 17a side of the shaft portion 17 around which the wire 2 is wound is closed off so that an operator's hand is not able to come into direct contact with the stent 5 which has been disassembled into the form of the wire 2. In addition, as is shown in FIG 8, it is also possible to construct a cover side aperture in which an insertion slit 18b which is wide enough to allow the sheath 7 to be fitted therein is provided extending in the axial direction of the cover side aperture.

The rotation operating portion 19 is formed in a bar shape, and is mounted in a direction substantially perpendicular to the center axis C of the shaft portion 17. A handle 19A which protrudes in the direction of the center axis C is provided at a distal end of the rotation operating portion 19.

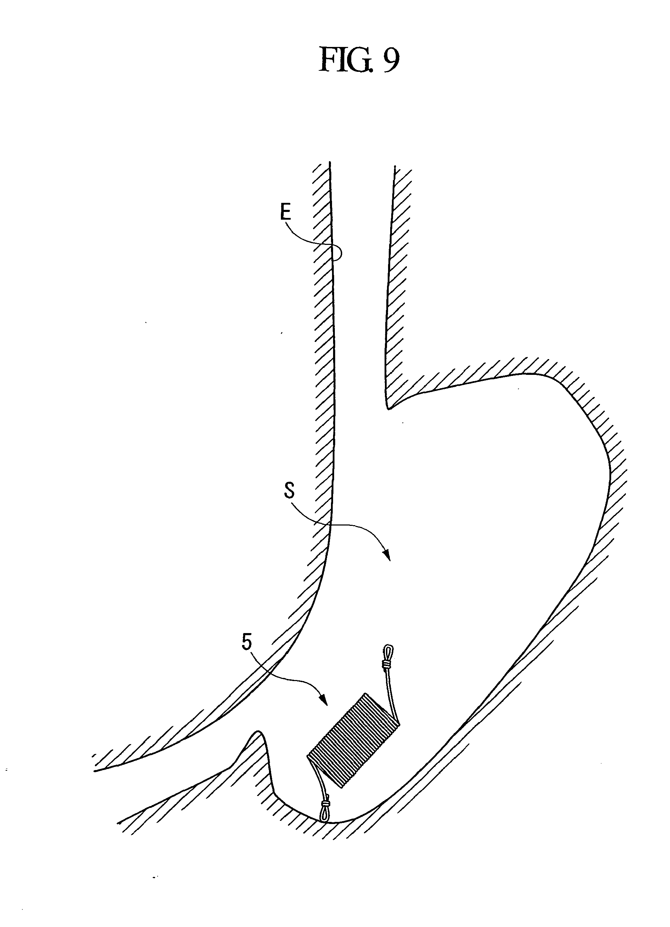

Next, using FIG. 9 through FIG. 17, a description will be given of the operation of the stent recovery apparatus 1 according to the present embodiment when a stent 5 that has been left in an esophagus E or that has fallen into a stomach is recovered. In the present embodiment, as is shown in FIG. 9, a description is given of when a stent 5 that has fallen into a stomach S is recovered.

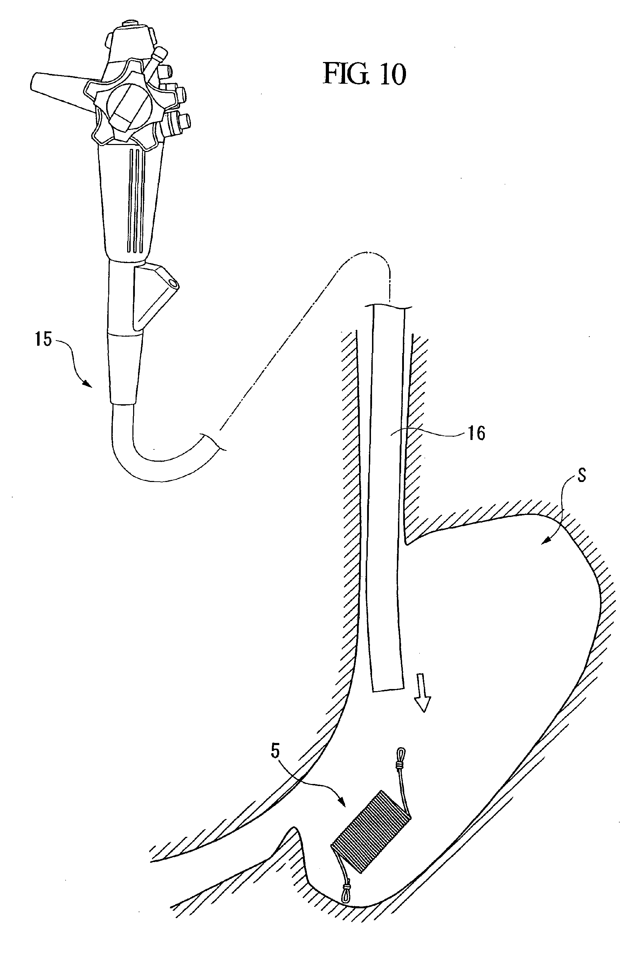

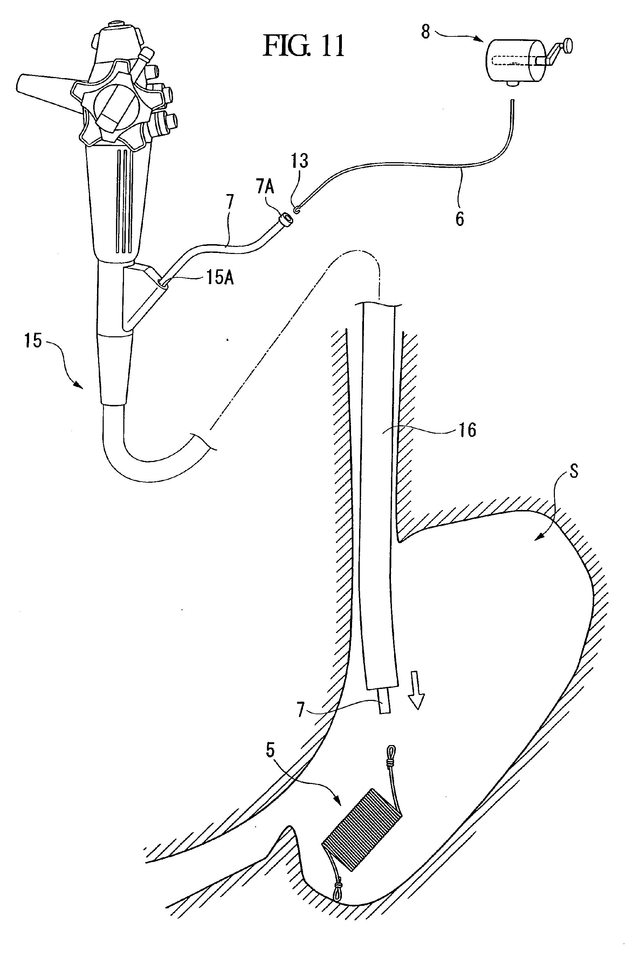

Firstly, as is shown in FIG 10, the insertion portion 16 of the endoscope 15 is inserted into the stomach S as far as the vicinity of the stent 5. In this state, as is shown in FIG. 11, the sheath 7 is inserted via a forceps aperture 15A into a treatment tool through channel (not shown) in the insertion portion 16. The traction wire 6 is then inserted into the sheath 7 via the sheath side aperture 7A.

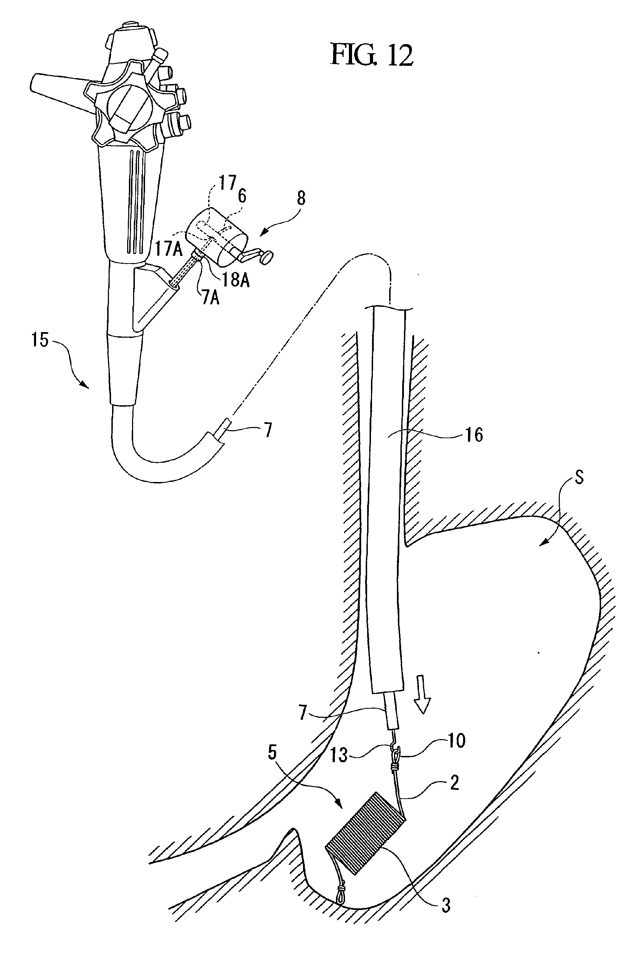

The hook 13 of the traction wire 6 is then made to protrude from the distal end of the sheath 7 and, while observing the operation using the endoscope 15, an operator operates the traction wire 6 so that the hook 13 is engaged with the catching portion 10 of the stent 5. In this state, as is shown in FIG 12, the traction wire 6 which is protruding from the base end of the sheath 7 is inserted through the through hole 18a of the cover portion 18, and is engaged with the engaging hole 17A in the shaft portion 17 of the retrieval tool 8. The sheath side aperture 7A and the cover side aperture 18A are then connected.

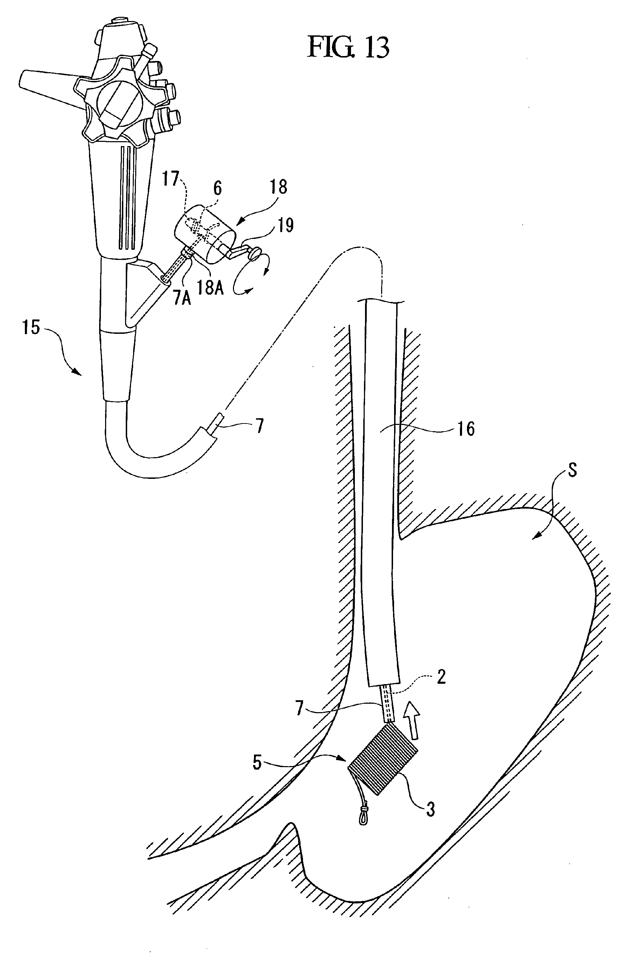

In this state, the operator grips the rotation operating portion 19 and rotates the shaft portion 17 around the center axis C of the shaft portion 17. At this time, as is shown in FIG 13, the traction wire 6 is wound around the shaft portion 17 from the base end side thereof and, in conjunction with this, the hook 13 that is connected to the distal end of the traction wire 6 becomes lodged within the sheath 7. At this time, the wire 2 is pulled so that the drum portion 3 becomes unwound and is pulled from the free end 2a side thereof into the sheath 7.

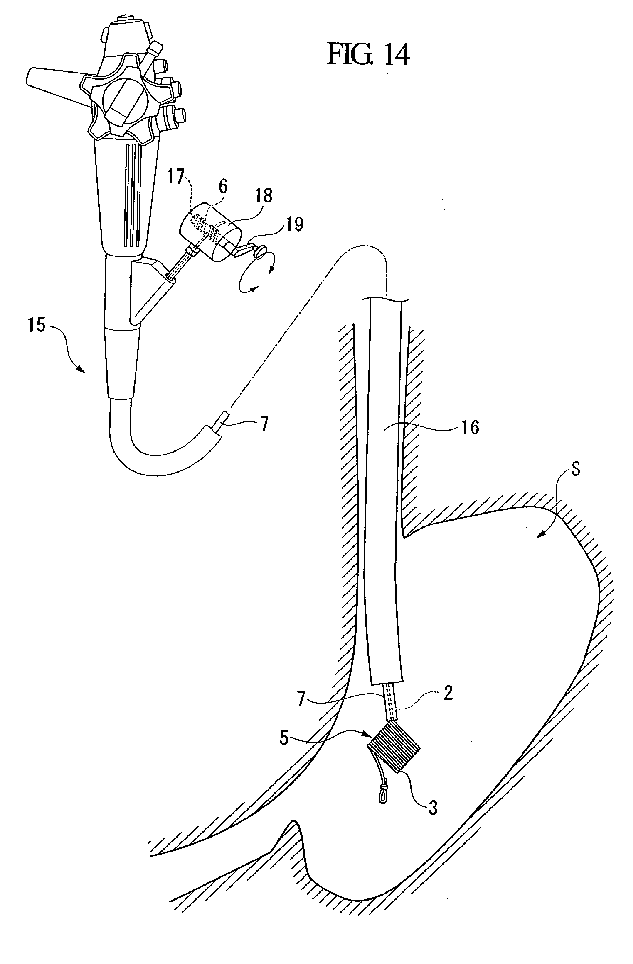

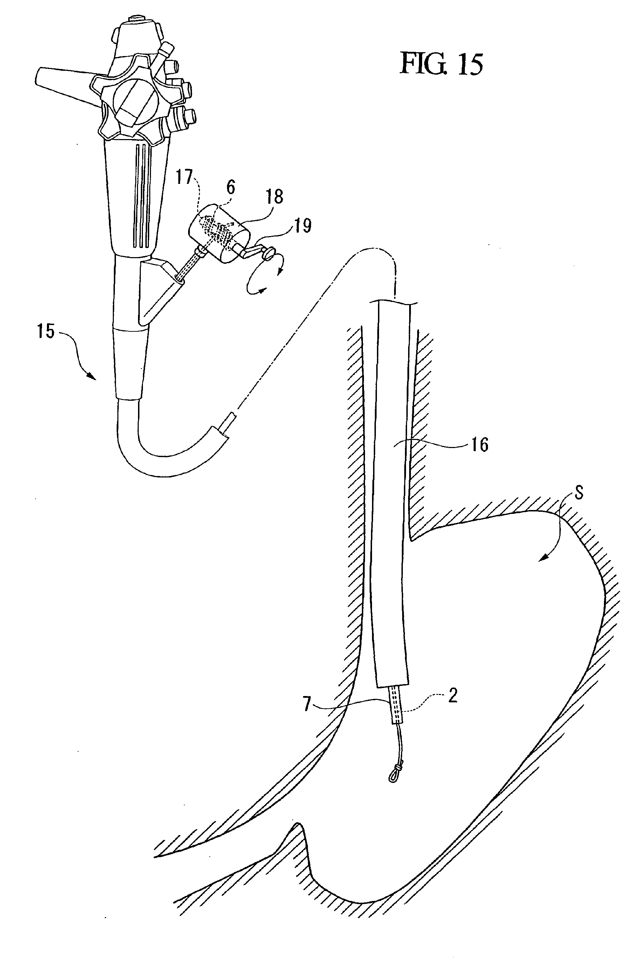

As the shaft portion 7 is rotated further, as is shown in FIG. 14, the wire 2 of the drum portion 3 is further unwound so that the axial length of the stent 5 becomes gradually shorter. In this manner, as is shown in FIG. 15, the stent 5 is completely unwound.

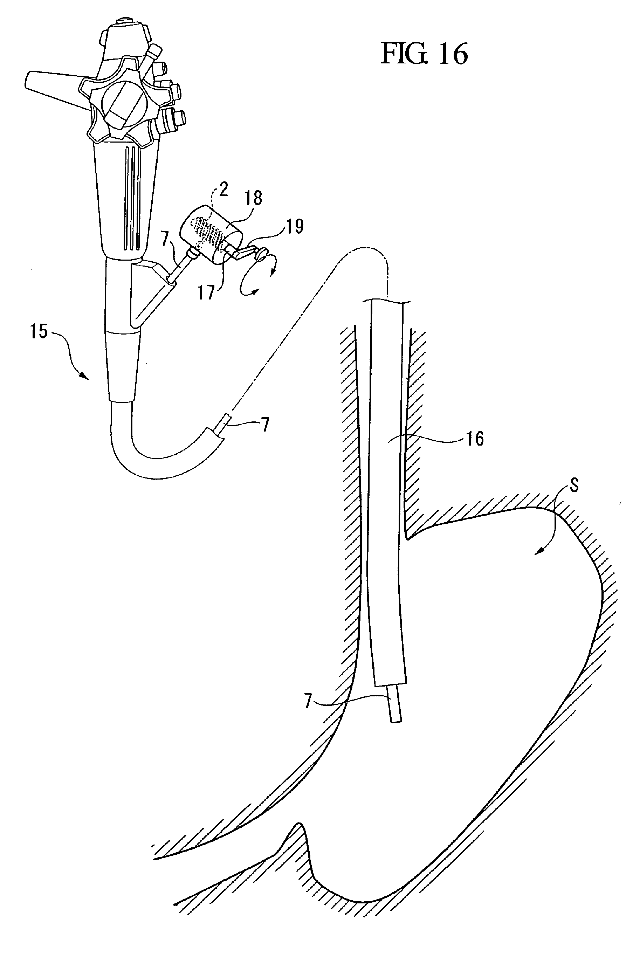

By further rotating the shaft portion 17, as is shown in FIG. 16, not only the traction wire 6, but also the wire 2 are wound onto the shaft portion 17.

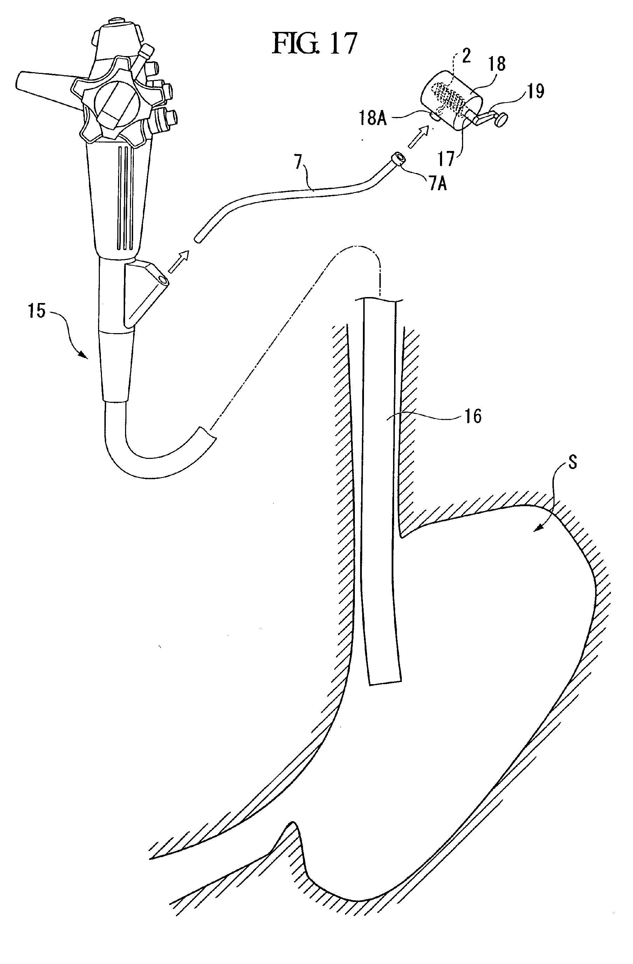

Ultimately, as is shown in FIG 17, the sheath side aperture 7A and the cover side aperture 18A are separated, and the sheath 7 is withdrawn via the treatment tool through channel in the insertion portion 16. In this manner, the stent 5 is recovered in the form of the wire 2.

According to this stent recovery apparatus 1, by pulling the free end 2a of the wire 2 of the drum portion 3 of the stent 5, the drum portion 3 can be unwound and the stent 5 restored to the state of a single wire 2. As a result, by gripping the catching portion 10 using the hook 13 of the traction wire 6, and retrieving the wire 2 using the retrieval tool 8 at the same time as the distal end of the sheath 7 is placed against the drum portion 3, the stent 5 can be recovered to the outside of the body as the drum portion 3 of the stent 5 is being unwound. Accordingly, it is possible to efficiently and rapidly recover the stent 5 to the outside of a body.

Moreover, because the traction wire 6 and the wire 2 of the stent 5 are moved through the interior of the sheath 7 during a recovery operation, it is possible to completely prevent an operator directly touching the wire 2 when the stent 5 is being recovered. Furthermore, because the one end 17a side of the shaft portion 17 onto which the wire 2 is wound is contained inside the cover portion 18 when the stent 5 is being recovered, it is possible here as well to completely prevent an operator directly touching the wire 2.

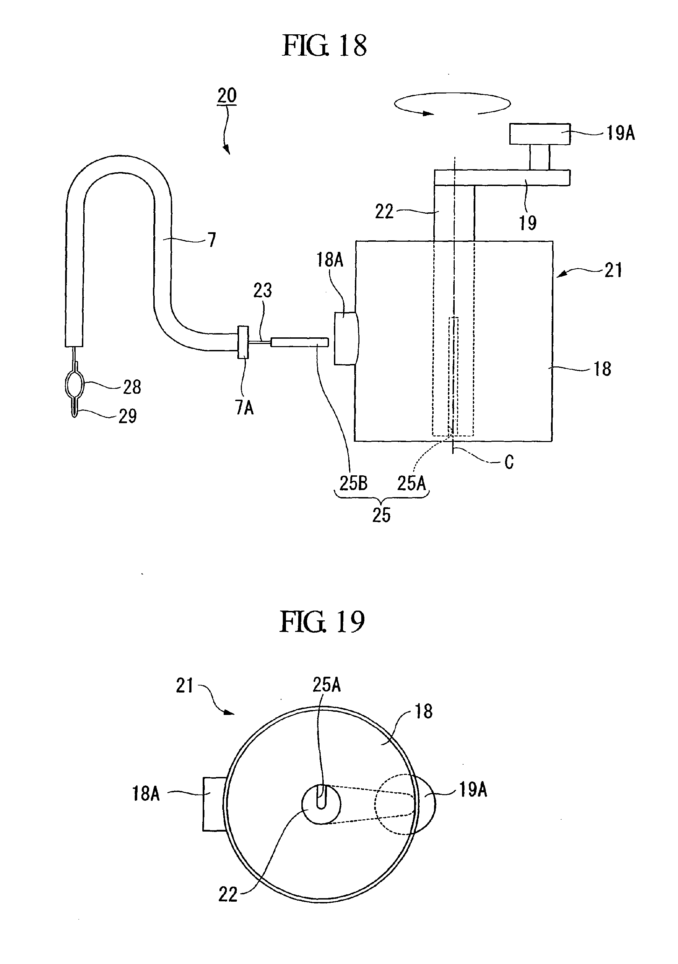

Next, a second embodiment of the present invention will be described with reference made to FIGS. 18 through 22.

Note that component elements that are the same as those of the above described first embodiment are given the same symbols and a description thereof is omitted.

The second embodiment differs from the first embodiment in that, as is shown in FIGS. 18 through 20, a stent recovery apparatus 20 according to the present embodiment comprises an engaging portion 25 with which a shaft portion 22 of a retrieval tool 21 and a base end of a traction wire 23 are engaged.

The engaging portion 25 comprises a groove portion 25A which runs along the center axis C of the shaft portion 22, and with a circular rod-shaped portion 25B that is provided at a base end of the traction wire 23 and has an outer diameter which enables it to fit into the groove portion 25A.

The length of the groove portion 25A is sufficient for the circular rod portion 25B to be fixed therein.

The outer diameter of the circular rod portion 25B is formed so as to be smaller than the inner diameters of the sheath 7 and the through hole 18a of the cover side aperture 18A.

A catching portion 27 of a stent 26 is formed in a spherical shape instead of a loop shape.

Instead of the hook 13, a snare loop 28 is formed by bending the traction wire 23 into a loop shape whose diameter is larger than the outer diameter of the sheath 7 at the distal end of the traction wire 23. A nose portion 29 is provided at the distal end of the snare loop 28 so as to protrude in the axial direction of the traction wire 23. This nose portion 29 is formed by folding back the traction wire 23 so as to form a gap between the adjacent traction wires 23 that is larger than the diameter of the wire 2 of the stent 26, but is smaller than the outer diameter of the catching portion 27.

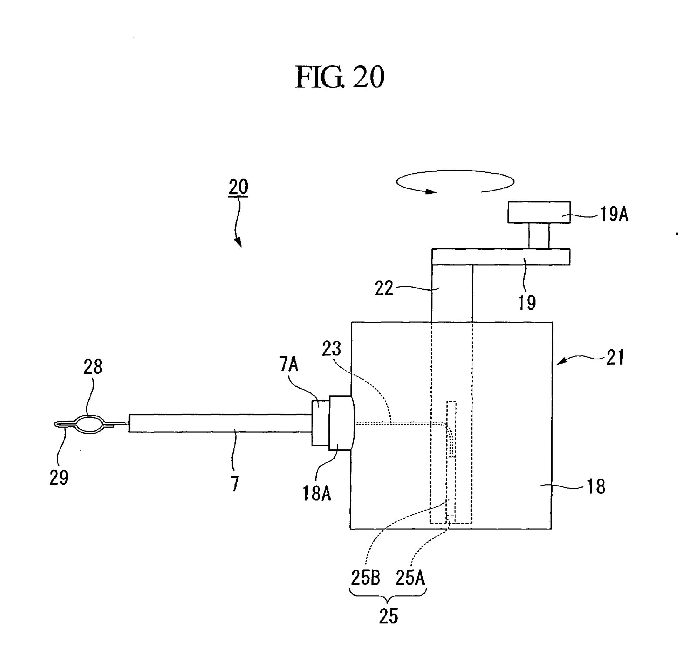

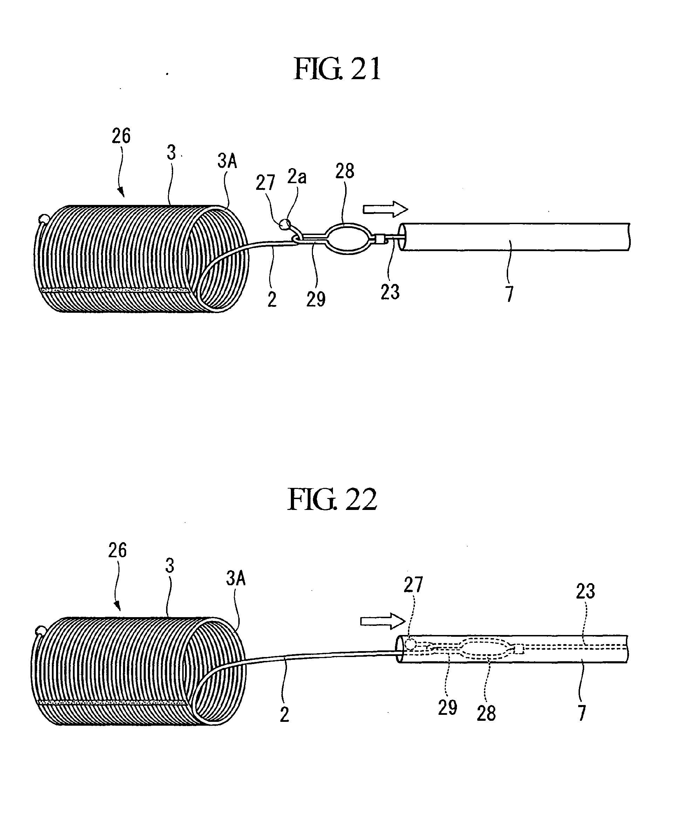

Next, a description will be given using FIG. 20 and FIG. 21 of the operation of the stent recovery apparatus 20 according to the present embodiment for a method in which a stent 26 which has fallen into a stomach or been left in an esophagus is recovered in the same way as in the first embodiment.

An insertion portion of an endoscope (not shown) is inserted into a stomach as far as the vicinity of the stent 26. In this state, the sheath 7 is inserted into a treatment tool through channel (not shown), and the traction wire 23 is then inserted into the sheath 7 via the sheath side aperture 7A.

The snare loop 28 of the traction wire 23 is then made to protrude from the distal end of the sheath 7 and, while observing the operation using the endoscope, an operator operates the traction wire 23 so that the catching portion 27 of the stent 26 is inserted through the snare loop 28. As is shown in FIG 21, the wire 2 is caught by the nose portion 29. In this state, the circular rod portion 25B of the traction wire 23 which is protruding from the base end of the sheath 7 is inserted through the cover side aperture 18A, and the circular rod portion 25B is fitted into the groove portion 25A which is provided in the shaft portion 22 of the retrieval tool 21. The sheath side aperture 7A and the cover side aperture 18A are then connected.

In this state, the operator grips the rotation operating portion 19 and rotates the shaft portion 22 around the center axis C of the shaft portion 22. At this time, in the same way as in the first embodiment, the traction wire 23 is wound around the shaft portion 22 and, as a result of the traction wire 23 being moved backwards relative to the sheath 7, as is shown in FIG. 22, the snare loop 28 is pulled into the sheath 7 while being contracted. At this time, the drum portion 3 of the stent 26 is unwound back to its state as the wire 2, and is pulled into the sheath 7 from the free end 2a side thereof.

As the shaft portion 22 is rotated further, in the same way as in the first embodiment, the stent 26 becomes completely unwound, and not only the traction wire 23, but the wire 2 as well are wound onto the shaft portion 22, thereby ending the recovery of the stent 26.

According to this stent recovery apparatus 20, when the wire 2 is being retrieved by the retrieval tool 21, it is possible to fix the retrieval direction of the traction wire 23 relative to the retrieval tool 21, and smoothly retrieve the traction wire 23.

Note that the range of the technology of the present invention is not limited to the above described embodiments and various modifications may be made thereto insofar as they do not depart from the spirit or scope of the present invention.

For example, in the above described first and second embodiments, it is also possible for the sheath and retrieval tool, and the traction wire and shaft portion to be connected together in advance.

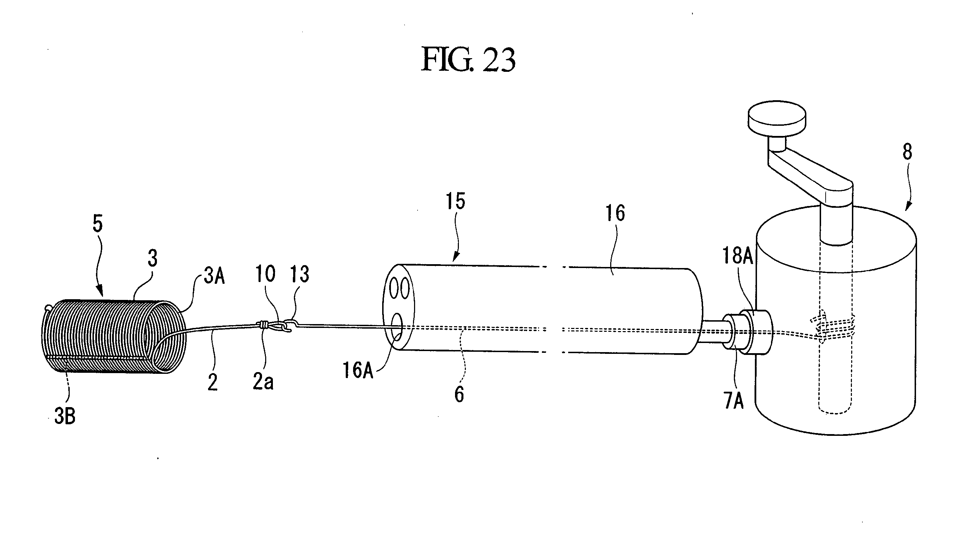

Moreover, in the first embodiment, a structure is employed in which the stent recovery apparatus 1 comprises the sheath 7, however, as is shown in FIG 23, it is also possible to make the treatment tool through channel 16A of the insertion portion 16 function as an insertion portion instead of the sheath 7, and use the traction wire 6 by inserting it directly into the treatment tool through channel 16A. In this case, by placing the distal end surface of the insertion portion 16 in direct contact with the drum portion 3 of the stent 5, the wire 2 can be pulled by the traction wire 6 into the treatment tool through channel 16A and the stent 5 can be recovered in the form of the wire 2 in the same way as in the first embodiment.

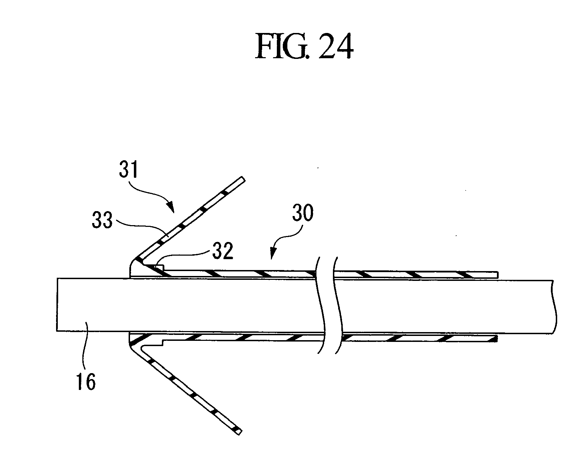

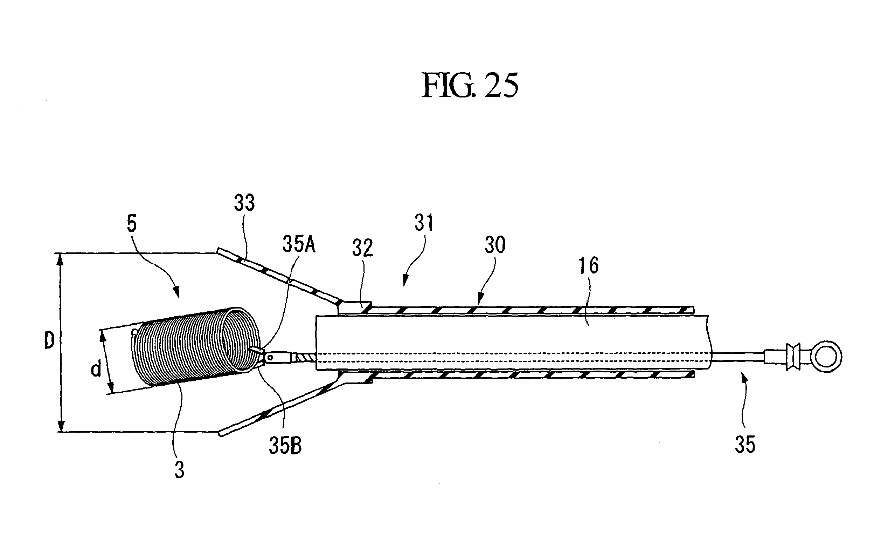

Moreover, as is shown in FIG. 24 and FIG. 25, it is also possible to employ a stent recovery apparatus 31 that comprises an overtube (i.e., a through portion) 30 whose inner diameter is substantially the same as the outer diameter of the insertion portion 16 of the endoscope 15. An increasing aperture portion 33 whose diameter becomes gradually wider towards the distal end thereof is provided via a flexible bending portion 32 at a distal end of this overtube 30. If the maximum inner diameter of this increasing aperture portion 33 is taken as D, and the outer diameter of the stent 5 is taken as d, then D ≧ d. The increasing aperture portion 33 has sufficient flexibility to allow it to be folded back towards the outer side in the radial direction using the bending portion 32 as a base point towards the base end side of the overtube 30.

This stent recovery apparatus 31 comprises gripping forceps 35 that has a pair of forcep pieces 35A and 35B and is able to be inserted through the insertion portion 16. The drum portion 3 of the stent 5 is able to be gripped by the pair of forcep pieces 35A and 35B.

An operation of this stent recovery apparatus 31 will now be described.

When the overtube 30 is inserted into an esophagus, the increasing aperture portion 33 at the distal end thereof is folded back towards the base end side of the overtube 30, and the overtube 30 is inserted such that the outer diameter of the distal end side thereof becomes gradually larger as it approaches the base end side. As a result, the overtube 30 can be inserted smoothly into the esophagus.

When recovering the stent 5, the stent 5 is gripped using the gripping forceps 35 that have been inserted through the treatment tool through channel 16A of the insertion portion 16, and the stent 5 is drawn into the increasing aperture portion 33 of the overtube 30 while the insertion portion 16 is being pulled just as it is towards the operator. While the stent 5 becomes even more deformed, the insertion portion 16 is then pulled further and is finally removed from the overtube 30. Alternatively, the insertion portion 16 may be removed together with the overtube 30. At this time, the increasing aperture portion 33 is bent by a stomach orifice (not shown) towards the distal end side of the overtube 30 once again using the bending portion 32 as a base point. As a result, because the distal end side of the overtube 30 once again becomes gradually larger in diameter from the base end side towards the distal end side thereof, in the same way as during insertion, it is able to transit smoothly through the esophagus.

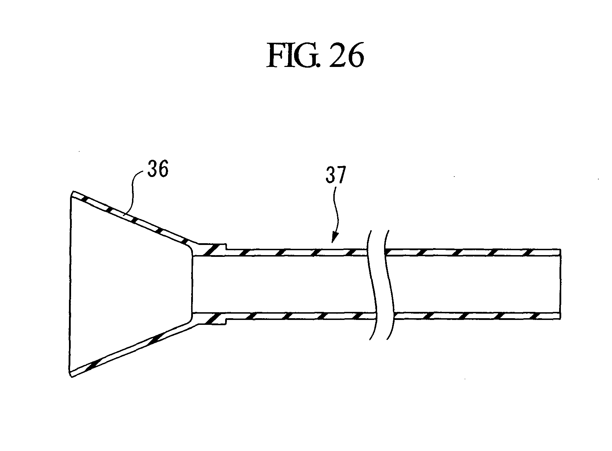

When there is only a small difference between the maximum inner diameter D of the increasing aperture portion 33 and the outer diameter d of the stent 5, then as is shown in FIG 26, it is also possible for an overtube 37 to be used in which an increasing aperture portion 36 is not folded back but has a fixed diameter.

As has been described above, according to the present invention, by pulling the free end of the wire or stranded wire of a drum portion of a stent using a traction tool, the drum portion can be unwound and the stent can be restored to the form of a single wire or strand wire. In addition, by gripping the wire or stranded wire using a traction tool and retrieving the wire or stranded wire using a retrieval tool while the insertion portion is placed against the drum portion, the stent can be recovered to outside the body while the drum portion of the stent is being unwound.

Moreover, according to the present invention, when a stent is being recovered, because the wire or stranded wire moves through the through portion, it is possible to completely prevent an operator from directly handling the moving wire or stranded wire.

Moreover, according to the present invention, when a stent is being recovered, because the shaft portion around which the wire or stranded wire is wound is housed inside a cover portion, it is possible to completely prevent an operator from directly handling the wire or stranded wire.

Moreover, according to the present invention, when the wire or stranded wire is being retrieved using a retrieval tool, it is possible to fix the direction in which the traction tool is retrieved relative to the retrieval tool, so that the traction tool can be retrieved smoothly.

Moreover, according to the present invention, a stent can be recovered by inserting a wire or stranded wire directly through a treatment tool through channel of an endoscope.

Moreover, according to the present invention, it is possible to efficiently and quickly recover a stent.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as limited by the foregoing description and is only limited by the scope of the appended claims.

## Claims

1. A stent recovery apparatus that recovers a stent which is left inside a body cavity and which comprises a hollow drum portion which is formed by a single wire or a stranded wire which has a free end, and which is disassembled by the wire or the stranded wire being unwound by being pulled, the stent recovery apparatus comprising:
a narrow elongated traction tool that catches the free end of the wire or stranded wire of the stent;
a through portion through which the traction tool and the wire or the stranded wire can be retrievably inserted, and whose distal end can be placed against the drum portion; and
a retrieval tool that retrieves the wire or the stranded wire which has been inserted through the through portion together with the traction tool.

2. The stent recovery apparatus according to claim 1, wherein the through portion is formed in a tube shape, and
a base end of the through portion is connected to the retrieval tool thereby connecting together the through portion and the retrieval tool.

3. The stent recovery apparatus according to claim 1, wherein the retrieval tool comprises:
a shaft portion around which the wire or stranded wire is wound; and
a cover portion that covers at least one end side of the shaft portion.

4. The stent recovery apparatus according to claim 1, further comprising an engaging portion that engages the retrieval tool with a base end of the traction tool.

5. The stent recovery apparatus according to claim 1, further comprising an endoscope that has an insertion portion in which a treatment tool through channel is provided, wherein the through portion is formed by the treatment tool through channel.
